# EUROPEAN PATENT SPECIFICATION

(11) **EP 1 924 687 B1**
(45) Date of publication and mention of the grant of the patent: **03.08.2011**
(21) Application number: 06798078.9
(22) Date of filing: 12.09.2006
(51) Int. Cl.: C12N 9/10, C12N 15/09, C12N 5/10, C12G 1/00, C12C 11/00

(54) **ALCOHOL ACETYL TRANSFERASE GENE AND USE THEREOF**
ALKOHOL-ACETYL-TRANSFERASEGEN UND DESSEN VERWENDUNG
GENE D'ALCOOL ACETYLE TRANSFERASE ET UTILISATION ASSOCIEE

(30) Priority: 13.09.2005 JP 2005266068; 24.07.2006 JP 2006200892
(43) Date of publication of application: 28.05.2008
(73) Proprietor: Suntory Holdings Limited, Kita-ku, Osaka-shi, Osaka 530-8203 (JP)
(72) Inventor: NAKAO, Yoshihiro, Mishima-gun, Osaka 618-8503 (JP); KODAMA, Yukiko, Mishima-gun, Osaka 618-8503 (JP); SHIMONAGA, Tomoko c/o Suntory Limited, Mishima-gun Osaka 6188503 (JP)
(74) Representative: Vossius & Partner
(86) International application number: PCT/JP2006/318467
(87) International publication number: WO 2007/032524

(56) References cited:
- JP-A- 2004 283 169
- VERSTREPEN KEVIN J ET AL: "Expression levels of the yeast alcohol acetyltransferase genes ATF1, Lg-ATF1, and ATF2 control the formation of a broad range of volatile esters." APPLIED AND ENVIRONMENTAL MICROBIOLOGY, vol. 69, no. 9, September 2003 (2003-09), pages 5228-5237, XP002421479 ISSN: 0099-2240 -& DATABASE EMBL 6 September 2003 (2003-09-06), "Saccharomyces cerevisiae CMBS212 alcohol acetyltransferase II (ATF2) gene, complete cds" XP002421491 Database accession no. AY242065 -& DATABASE UniProt 5 July 2004 (2004-07-05), "Alcohol acetyltransferase /EC 2.3.1.84) (Alcohol acetyltransferase II)" XP002421492 Database accession no. Q7LIK2
- DATABASE GENESEQ 22 April 2004 (2004-04-22), "Yeast alcohol acetyltransferase, ATF2, coding sequence" XP002421493 Database accession no. ADI57374 -& DATABASE GENESEQ 22 April 2004 (2004-04-22), "Yeast alcohol acetyltransferase, ATF2" XP002421494 Database accession no. ADI57375 -& EP 1 308 501 A (PERNOD RICARD [FR]) 7 May 2003 (2003-05-07)
- DATABASE GENESEQ 10 February 1997 (1997-02-10), "Alcohol acetyltransferase coding sequence from sake yeast" XP002421495 Database accession no. AAT43690 -& DATABASE GENESEQ 10 February 1997 (1997-02-10), "Alcohol acetyltransferase form sake yeast" XP002421496 Database accession no. AAW06744 -& JP 08 242851 A (OZEKI KK; KIRIN BREWERY) 24 September 1996 (1996-09-24)
- YOSHIMOTO HIROYUKI ET AL: "Isolation and characterization of the ATF2 gene encoding alcohol acetyltransferase II in the bottom fermenting yeast Saccharomyces pastorianus" YEAST, vol. 15, no. 5, 30 March 1999 (1999-03-30), pages 409-417, XP002421481 ISSN: 0749-503X -& DATABASE EMBL 29 July 1996 (1996-07-29), "Saccharomyces pastorianus ATF2 gene for alcohol acetyltransferase, complete cds" XP002421497 Database accession no. D86480 -& DATABASE UniProt 1 November 1996 (1996-11-01), "Alcohol acetyltransferase" XP002421498 Database accession no. Q12304

### SEQUENCE LISTING

<110> Suntory Limited
<120> Alcohol acetyl transferase gene and use thereof
<130> PCT06-0082
<150> JP2005-266068
   <151> 2005-09-13
<150> JP2006-200892
   <151> 2006-07-24
<160> 8
<170> Patentin version 3.3
<210> 1
   <211> 1638
   <212> DNA
   <213> Saccharomyces sp.
<400> 1
   atggatagtt tagaggaata cgaaccgcac gttacgcaag agctgatcga tcgtggccat     60
   gcaaggcgca tgggccattt ggaaaactat tttgctgttt tgcgtaggca aaacatgtat    120
   tcgaatttcg ctgtttatgg ggaactgaat aaggccgtta gcaagagaca actgagactt    180
   gccttgaggt tattgcttct aaagtatcca attctttcgc atacaattgt tcccaaaaaa    240
   taccctaacc acgaagcgta ttatctcagt caggaatatc tgaataagcc gtttcctcag    300
   catgatttta tcaaggttat acctcaccta gaactggagg atttaatcat gaacagtcaa    360
   tcagaataca gggaagtcat cggcaaaatt tcggaacaat ataagaatga taattttaaa    420
   gtcacgagta gcttaatcga attagtcgct cctataatcg taccggcatg tgacccggaa    480
   aggcccaatt ggaggcttat ttgtttacct agtagggata ctagcgcggg cgaggcgtgg    540
   accaacttcg tttatgtcag taaccattgc ggctccgatg gtattagtgg aacgaatttt    600
   tttcaggatt tgactgcttt actctctacg atcgaggaga acgggttcga ctatgaggag    660
   gagatcgtgg ataacaacgt catcatcgac tataacgaag atcacaaaga aatctcgaag    720
   cttccgatct ccatcacaga ccgtattgac tataagccta gattgacgtc gctacctaaa 780
   ttcttcttga agaacatcgt gtctgagtat tgtgagttca gaacttccaa tgagtcgaca 840
   gtcacttcca ggtactctcc ctcctctaat gcaaatgcag gtttcaacca tctgttgcat 900
   ttcagtgccg aagatacaca acaaattagg tcggaaatta aaaaaaaagt tcatgctgga 960
   tgtaccataa cgcccttcat tcaagcctgc tttttcgtag ctttatatag acacgataaa 1020
   gttttcacca agtccttcct tgaatatggg ttcgacgtgg ccctcccaag tagcacaaga 1080
   agattcttac caaacgacga agagctaaga gactcctaca aatacggcgc aaacgttggt 1140
   ggttcacatt acacctattt gatctcctct tttaacgtcc ccgagggcga cgccaacaag 1200
   ttctggcgtc tcgtcgagta ctaccacgac tgcttcttaa agtcgtacga taacggagac 1260
   cacttgatcg gcttgggcac actacaacta gacttcatcg ttcaaaacaa gaacgtggac 1320
   caaattattt ccaccaccta cttggaccac caaagaggcg gtgccatcat cagcaacaca 1380
   gggtttgtca ggcaagatac gaccaagccg tactacgccc gcgacctaat attctcacag 1440
   tctgcaggcg ccttgagatt tgcgtttggc ctaaacgtct gctccacaaa cgttggtggt 1500
   atgaacatgg acatgagcgt agttcagggc actctacgga atcggggcga atgggagtcg 1560
   ttctgcgacg ttttctacca cgccatcggc gaatttgcgt cgcttggatg cgatactgcc 1620
   gtcgcgggtc tcgtatag 1638
<210> 2
   <211> 545
   <212> PRT
   <213> Saccharomyces sp.
<400> 2
   Met Asp Ser Leu Glu Glu Tyr Glu Pro His Val Thr Gln Glu Leu Ile
   1 5 10 15
   Asp Arg Gly His Ala Arg Arg Met Gly His Leu Glu Asn Tyr Phe Ala
   20 25 30
   Val Leu Arg Arg Gln Asn Met Tyr Ser Asn Phe Ala Val Tyr Gly Glu
           35                  40                  45
   Leu Asn Lys Ala Val Ser Lys Arg Gln Leu Arg Leu Ala Leu Arg Leu
       50                  55                  60
   Leu Leu Leu Lys Tyr Pro Ile Leu Ser His Thr Ile Val Pro Lys Lys
   65                  70                  75                  80
   Tyr Pro Asn His Glu Ala Tyr Tyr Leu Ser Gln Glu Tyr Leu Asn Lys
                   85                  90                  95
   Pro Phe Pro Gln His Asp Phe Ile Lys Val Ile Pro His Leu Glu Leu
               100                 105                 110
   Glu Asp Leu Ile Met Asn Ser Gln Ser Glu Tyr Arg Glu Val Ile Gly
           115                 120                 125
   Lys Ile Ser Glu Gln Tyr Lys Asn Asp Asn Phe Lys Val Thr Ser Ser
       130                 135                 140
   Leu Ile Glu Leu Val Ala Pro Ile Ile Val Pro Ala Cys Asp Pro Glu
   145                 150                 155                 160
   Arg Pro Asn Trp Arg Leu Ile Cys Leu Pro Ser Arg Asp Thr Ser Ala
                   165                 170                 175
   Gly Glu Ala Trp Thr Asn Phe Val Tyr Val Ser Asn His Cys Gly Ser
               180                 185                 190
   Asp Gly Ile Ser Gly Thr Asn Phe Phe Gln Asp Leu Thr Ala Leu Leu
           195                 200                 205
   Ser Thr Ile Glu Glu Asn Gly Phe Asp Tyr Glu Glu Glu Ile Val Asp
   210 215 220
   Asn Asn Val Ile Ile Asp Tyr Asn Glu Asp His Lys Glu Ile Ser Lys
   225 230 235 240
   Leu Pro Ile Ser Ile Thr Asp Arg Ile Asp Tyr Lys Pro Arg Leu Thr
   245 250 255
   Ser Leu Pro Lys Phe Phe Leu Lys Asn Ile Val Ser Glu Tyr Cys Glu
   260 265 270
   Phe Arg Thr Ser Asn Glu Ser Thr Val Thr Ser Arg Tyr Ser Pro Ser
   275 280 285
   Ser Asn Ala Asn Ala Gly Phe Asn His Leu Leu His Phe Ser Ala Glu
   290 295 300
   Asp Thr Gln Gln Ile Arg Ser Glu Ile Lys Lys Lys Val His Ala Gly
   305 310 315 320
   Cys Thr Ile Thr Pro Phe Ile Gln Ala Cys Phe Phe Val Ala Leu Tyr
   325 330 335
   Arg His Asp Lys Val Phe Thr Lys Ser Phe Leu Glu Tyr Gly Phe Asp
   340 345 350
   Val Ala Leu Pro Ser Ser Thr Arg Arg Phe Leu Pro Asn Asp Glu Glu
   355 360 365
   Leu Arg Asp Ser Tyr Lys Tyr Gly Ala Asn Val Gly Gly Ser His Tyr
   370 375 380
   Thr Tyr Leu Ile Ser Ser Phe Asn Val Pro Glu Gly Asp Ala Asn Lys
   385 390 395 400
   Phe Trp Arg Leu Val Glu Tyr Tyr His Asp Cys Phe Leu Lys Ser Tyr
   405 410 415
   Asp Asn Gly Asp His Leu Ile Gly Leu Gly Thr Leu Gln Leu Asp Phe
   420 425 430
   Ile Val Gln Asn Lys Asn Val Asp Gln Ile Ile Ser Thr Thr Tyr Leu
   435 440 445
   Asp His Gln Arg Gly Gly Ala Ile Ile Ser Asn Thr Gly Phe Val Arg
   450 455 460
   Gln Asp Thr Thr Lys Pro Tyr Tyr Ala Arg Asp Leu Ile Phe Ser Gln
   465 470 475 480
   Ser Ala Gly Ala Leu Arg Phe Ala Phe Gly Leu Asn Val Cys Ser Thr
   485 490 495
   Asn Val Gly Gly Met Asn Met Asp Met Ser Val Val Gln Gly Thr Leu
   500 505 510
   Arg Asn Arg Gly Glu Trp Glu Ser Phe Cys Asp Val Phe Tyr His Ala
   515 520 525
   Ile Gly Glu Phe Ala Ser Leu Gly Cys Asp Thr Ala Val Ala Gly Leu
   530 535 540
   Val
   545
<210> 3
   <211> 40
   <212> DNA
   <213> Artificial
<220>
   <223> nonScATF2_for
<400> 3
   gagctcatag cggccatgga tagtttagag gaatacgaac 40
<210> 4
   <211> 42
   <212> DNA
   <213> Artificial
<220>
   <223> nonScATF2_rv
<400> 4
   ggatcctatg cggccgcacc gaccgagagc agccacggca tg 42
<210> 5
   <211> 70
   <212> DNA
   <213> Artificial
<220>
   <223> nonScATF2_delta_for
<400> 5
   ttaagatcta caaacgaaca ataggatcaa gaacagaaaa ctgcagtgaa ccttgacagt 60
   cttgacgtgc 70
<210> 6
   <211> 70
   <212> DNA
   <213> Artificial
<220>
   <223> nonScATF2_delta_rv
<400> 6
   tatcagctct gtccgatata ccgcctattt gctgtacgag cccgtccgag cgcacttaac 60
   ttcgcatctg 70
<210> 7
   <211> 70
<212> DNA
   <213> Artificial
<220>
   <223> nonScEHT1_delta_for
<400> 7
   ggccggggaa gtaatagtcc cttcgaagat tttccttact atttaacagt ccttgacagt 60
   cttgacgtgc 70
<210> 8
   <211> 70
   <212> DNA
   <213> Artificial
<220>
   <223> nonScEHT1_delta _rv
<400> 8
   cttctgttgt atacatacat aagaatggct aggaaaagaa ccatataaca cgcacttaac 60
   ttcgcatctg 70

## Description

### TECHNICAL FIELD

The present invention relates to an alcohol acetyl transferase gene and to uses of the gene. The invention relates in particular to a brewer's yeast which produces alcoholic beverages with excellent aroma and flavor, alcoholic beverages produced using such a yeast, and a method of producing such alcoholic beverages. More specifically, the invention relates to an ATF2 gene which codes for the alcohol acetyl transferase Atf2p in brewer's yeast, particularly to a yeast whose capability of producing ester, which contribute to aroma and flavor of a product, is controlled by regulating the level of expression of the nonScA TF2 gene characteristic to beer yeast and to a method of producing alcoholic beverages using such a yeast.

### BACKGROUND ART

Esters are an important aromatic component of alcoholic beverages. In the case of rice wine, wine and whiskey, an increase in the ester content is known to give the beverage a florid aroma as well as cause it to be evaluated highly for its flavor. On the other hand, although esters are an important aromatic component of beer as well, an excess amount of esters is disliked due to the resulting ester smell. Thus, it is important to suitably control the amount of ester formed according to the type of alcoholic beverage.

Yeast producing high levels of esters have been developed in the past for the purpose of increasing the ester content of alcoholic beverages. Examples of previously reported methods for effective isolation of yeast producing large amounts of esters include a method in which yeast is subjected (or not subjected) to mutagenic treatment to obtain a strain which produces large amounts of caproic acid and is resistant to drugs that inhibit fatty acid synthases such as cerulenin, as well as a strain which is resistant to leucine analogs such as 5,5,5-trifluoro-DL-leucine and produces large amounts of isoamyl alcohol and isoamyl acetate (Japanese Patent Laid-open Publication No. 2002-253211), and a method in which a strain is acquired which is grown in medium containing a steroid having a pregnane backbone hydroxylated at position 3 (Japanese Patent Laid-open Publication No. 2002-191355).

On the other hand, examples of previously reported methods involving the development of yeast utilizing genetic engineering techniques include expressing high levels of the alcohol acetyl transferase gene ATF1 of Saccharomyces cerevisiae in brewing yeast (Japanese Patent Laid-open Publication No. H06-062849), inhibiting the expression of ATF1 (Japanese Patent Laid-open Publication No. H06-253826), and increasing the amount of ester by destroying esterase gene EST2 in brewing yeast (Japanese Patent Laid-open Publication No. H09-234077).

Verstrepen et al. (Appl. Environ. Microbiol. 69 (2003), 5228-5237), EP-A 1 1 308 501 and JP 08242851 describe the ATF2 of S. cerevisiae and its effect on the production of volatile esters. Yoshimoto et al. (Yeast 15 (1999), 409-417) reports on the isolation of an ATF2 gene from S. pastorianus which is highly similar to that of S. cerevisiae.

### DISCLOSURE OF INVENTION

As stated above, although a mutant strain is acquired for increasing the ester content of a product, there are cases in which unexpected delays in fermentation or increases in undesirable aromatic and flavor components are observed as a result thereof, thus creating problems in the development of yeast for practical application. Consequently, there is a need for a method for breeding yeast capable of producing a desired amount of esters without impairing the fermentation rate or product quality.

As a result of conducting extensive studies to solve the above-mentioned problems, the inventors of the present invention succeeded in identifying and isolating a gene from brewer's yeast which encodes an alcohol acetyl transferase. The inventors of the present invention confirmed that the amount of ester formed increases by producing a transformed yeast by inserting and expressing the resulting gene in yeast, thereby leading to completion of the present invention.

Namely, the present invention relates to the embodiments as specified in the claims. More specifically, the present invention provides the polynucleotide indicated below, a vector containing said polynucleotide, a transformed yeast in which said vector has been inserted, and a method for producing an alcoholic beverage using said transformed yeast. (1) A polynucleotide selected from the group consisting of:
(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one to 6 amino acids thereof being deleted, substituted, inserted and/or added, and having an alcohol acetyl transferase activity; and
(d) a polynucleotide comprising a polynucleotide encoding a protein having an amino acid sequence having 97% or higher identity with the amino acid sequence of SEQ ID NO:2, and having an alcohol acetyl transferase activity.
   (2) The polynucleotide of (1) above selected from the group consisting of:
(g) a polynucleotide comprising a polynucleotide encoding a protein consisting of the amino acid sequence of SEQ ID NO: 2, or encoding an amino acid sequence of SEQ ID NO: 2 wherein 1 to 2 amino acids thereof are deleted, substituted, inserted, and/or added, and wherein said protein has an alcohol acetyl transferase activity;
(h) a polynucleotide comprising a polynucleotide encoding a protein having 99% or higher identity with the amino acid sequence of SEQ ID NO: 2, and having an alcohol acetyl transferase activity.
   (3) The polynucleotide of (1) above comprising a polynucleotide consisting of the nucleotide sequence of SEQ ID NO: 1.
   (4) The polynucleotide of (1) above comprising a polynucleotide encoding a protein consisting of the amino acid sequence of SEQ ID NO: 2.
   (5) The polynucleotide of any one of (1) to (4) above, wherein the polynucleotide is DNA.
   (6) A polynucleotide encoding RNA of a nucleotide sequence complementary to a transcript of the polynucleotide (DNA) according to (5) above.
   (7) A protein encoded by the polynucleotide of any one of (1) to (5) above.
   (8) A vector comprising the polynucleotide of any one of (1) to (5) above.
   (9) A vector comprising the polynucleotide of (6) above.
   (10) A yeast, wherein the vector of (8) or (9) above is introduced.
   (11) The yeast of (10) above, wherein ester-producing ability is increased by introducing the vector of (8) above.
   (12) A yeast, wherein an expression of the polynucleotide (DNA) of (5) above is repressed by introducing the vector of (9) above, or by disrupting a gene related to the polynucleotide (DNA) of (5) above.
   (13) The yeast of (11) above, wherein a ester-producing ability is increased by increasing an expression level of the protein of (7) above.
   (14) A method for producing an alcoholic beverage by using the yeast of any one of (10) to (13) above.
   (15) The method for producing an alcoholic beverage of (14) above, wherein the brewed alcoholic beverage is a malt beverage.
   (16) The method for producing an alcoholic beverage of (14) above, wherein the brewed alcoholic beverage is a wine.
   (17) A method for assessing a test yeast for its ester-producing capability, comprising using a primer or a probe including part or all of the nucleotide sequence of an alcohol acetyl transferase gene having the nucleotide sequence of SEQ ID NO: 1 or including a nucleotide sequence complementary to said nucleotide sequence.
   (18) A method for assessing a test yeast for its ester-producing capability, comprising: culturing a test yeast; and measuring an expression level of an alcohol acetyl transferase gene having the nucleotide sequence of SEQ ID NO: 1.
   (19) A method for selecting a yeast, comprising: culturing test yeasts; quantifying the protein of (7) above or measuring an expression level of an alcohol acetyl transferase gene having the nucleotide sequence of SEQ ID NO: 1; and selecting a test yeast having said protein amount or said gene expression level according to a target capability of producing ester.
   (20) The method for selecting a yeast of (19) above, comprising: culturing a reference yeast and test yeasts; measuring an expression level of an alcohol acetyl transferase gene having the nucleotide sequence of SEQ ID NO: 1 in each yeast; and selecting a test yeast having the gene expressed higher or lower than that in the reference yeast.
   (21) The method for selecting a yeast of (19) above comprising: culturing a reference yeast and test yeasts; quantifying the protein of (7) above in each yeast; and selecting a test yeast having said protein for a larger amount than that in the reference yeast. That is, the method for selecting a yeast of (19) above comprising: culturing plural yeasts; quantifying the protein of (7) above in each yeast; and selecting a test yeast having a large amount of the protein from them. That is, the method for selecting a yeast of (19) above comprising: culturing plural yeasts; quantifying the protein of (7) above in each yeast; and selecting a test yeast producing a large or small amount of the protein from them.
   (22) A method for producing an alcoholic beverage comprising: conducting fermentation for producing an alcoholic beverage using the yeast according to any one of (10) to (13) or a yeast selected by the method according to any one of (19) to (21); and adjusting the production amount of ester.

According to the method for producing alcohols by using the transformed yeast of the invention, ester contents can be controlled so that alcohols with enhanced flavor can be produced.

### BRIEF DESCRIPTION OF DRAWINGS

Figure 1 shows the cell growth with time upon beer fermentation test. The horizontal axis represents fermentation time while the vertical axis represents optical density at 660 nm (OD660).
Figure 2 shows the extract (sugar) consumption with time upon beer fermentation test. The horizontal axis represents fermentation time while the vertical axis represents apparent extract concentration (w/w%).
Figure 3 shows the expression profile of nonScATF2 gene in yeasts upon beer fermentation test. The horizontal axis represents fermentation time while the vertical axis represents the intensity of detected signal.
Figure 4 shows the cell growth with time upon fermentation test. The horizontal axis represents fermentation time while the vertical axis represents optical density at 660 nm (OD660).
Figure 5 shows the extract (sugar) consumption with time upon beer fermentation test. The horizontal axis represents fermentation time while the vertical axis represents apparent extract concentration (w/w%).

### BEST MODES FOR CARRYING OUT THE INVENTION

The present inventors conceived that it is possible to control ester in products by increasing or decreasing the disclosed alcohol acetyl transferase activity of the yeast. The present inventors isolated and identified a nonScATF2 gene encoding an alcohol acetyl transferase unique to lager brewing yeast based on the lager brewing yeast genome information mapped according to the method disclosed in Japanese Patent Laid-Open Publication No. 2004-283169. The nucleotide sequence of the gene is represented by SEQ ID NO: 1. Further, an amino acid sequence of a protein encoded by the gene is represented by SEQ ID NO: 2.

### 1. Polynucleotide of the invention

First of all, the present invention provides (a) a polynucleotide comprising a polynucleotide consisting of the nucleotide sequence of SEQ ID NO: 1; and (b) a polynucleotide comprising a polynucleotide encoding a protein of the amino acid sequence of SEQ ID NO:2. The polynucleotide can be DNA or RNA.

The target polynucleotide of the present invention is not limited to the polynucleotide encoding an alcohol acetyl transferase gene derived from lager brewing yeast described above and may include other polynucleotides encoding proteins having equivalent functions to said protein. Proteins with equivalent functions include, for example, (c) a protein of an amino acid sequence of SEQ ID NO: 2 with one to 6 amino acids thereof being deleted, substituted, inserted and/or added and having an alcohol acetyl transferase activity.

Such proteins include a protein consisting of an amino acid sequence of SEQ ID NO: 2 with 1 to 6 (1 to several amino acids), 1 to 5, 1 to 4, 1 to 3, 1 to 2, or 1 amino acid residues thereof being deleted, substituted, inserted and/or added and having an alcohol acetyl transferase activity. In general, the number of deletions, substitutions, insertions, and/or additions is preferably smaller. In addition, such proteins include (d) a protein having an amino acid sequence with 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher, or 99.9% or higher identity with the amino acid sequence of SEQ ID NO: 2, and having an alcohol acetyl transferase activity. In general, the percentage identity is preferably higher.

Alcohol acetyl transferase activity may be measured, for example, by a method described in Japanese Laid-open Patent Publication No. 253826.

The present invention also describes (e) a polynucleotide comprising a polynucleotide which hybridizes to a polynucleotide consisting of a nucleotide sequence complementary to the nucleotide sequence of SEQ ID NO: 1 under stringent conditions and which encodes a protein having an alcohol acetyl transferase activity; and (f) a polynucleotide comprising a polynucleotide which hybridizes to a polynucleotide complementary to a nucleotide sequence of encoding a protein of SEQ ID NO: 2 under stringent conditions, and which encodes a protein having an alcohol acetyl transferase activity.

Herein, "a polynucleotide that hybridizes under stringent conditions" refers to nucleotide sequence, such as a DNA, obtained by a colony hybridization technique, a plaque hybridization technique, a southern hybridization technique or the like using all or part of polynucleotide of a nucleotide sequence complementary to the nucleotide sequence of SEQ ID NO: 1 or polynucleotide encoding the amino acid sequence of SEQ ID NO: 2 as a probe. The hybridization method may be a method described, for example, in MOLECULAR CLONING 3rd Ed., CURRENT PROTOCOLS IN MOLECULAR BIOLOGY, John Wiley & Sons 1987-1997, and so on.

The term "stringent conditions" as used herein may be any of low stringency conditions, moderate stringency conditions or high stringency conditions. "Low stringency conditions" are, for example, 5 x SSC, 5 x Denhardt's solution, 0.5% SDS, 50% formamide at 32°C. "Moderate stringency conditions" are, for example, 5 x SSC, 5 x Denhardt's solution, 0.5% SDS, 50% formamide at 42°C. "High stringency conditions" are, for example, 5 x SSC, 5 x Denhardt's solution, 0.5% SDS, 50% formamide at 50°C. Under these conditions, a polynucleotide, such as a DNA, with higher homology is expected to be obtained efficiently at higher temperature, although multiple factors are involved in hybridization stringency including temperature, probe concentration, probe length, ionic strength, time, salt concentration and others, and one skilled in the art may appropriately select these factors to realize similar stringency.

When a commercially available kit is used for hybridization, for example, Alkphos Direct Labeling Reagents (Amersham Pharmacia) may be used. In this case, according to the attached protocol, after incubation with a labeled probe overnight, the membrane is washed with a primary wash buffer containing 0.1% (w/v) SDS at 55°C, thereby detecting hybridized polynucleotide, such as DNA.

Polynucleotides that hybridized are polynucleotides having 97% or higher, 98% or higher, 99% or higher, 99.1% or higher, 99.2% or higher, 99.3% or higher, 99.4% or higher, 99.5% or higher, 99.6% or higher, 99.7% or higher, 99.8% or higher or 99.9% or higher identity to polynucleotide encoding the amino acid sequence of SEQ ID NO: 2 as calculated by homology search software, such as FASTA and BLAST using default parameters.

Identity between amino acid sequences or nucleotide sequences may be determined using algorithm BLAST by Karlin and Altschul (*Proc. Natl. Acad. Sci. USA,* 87: 2264-2268, 1990; *Proc. Natl. Acad. Sci. USA,* 90: 5873, 1993). Programs called BLASTN and BLASTX based on BLAST algorithm have been developed (Altschul SF et al., *J*. *Mol. Biol.* 215: 403, 1990). When a nucleotide sequence is sequenced using BLASTN, the parameters are, for example, score = 100 and word length = 12. When an amino acid sequence is sequenced using BLASTX, the parameters are, for example, score = 50 and word length = 3. When BLAST and Gapped BLAST programs are used, default parameters for each of the programs are employed.

The polynucleotide of the present invention includes (j) a polynucleotide encoding RNA having a nucleotide sequence complementary to a transcript of the polynucleotide (DNA) according to (5) above. Also disclosed is: (k) a polynucleotide encoding RNA that represses the expression of the polynucleotide (DNA) according to (5) above through RNAi effect; (1) a polynucleotide encoding RNA having an activity of specifically cleaving a transcript of the polynucleotide (DNA) according to (5) above; and (m) a polynucleotide encoding RNA that represses expression of the polynucleotide (DNA) according to (5) above through co-supression effect. These polynucleotides may be incorporated into a vector, which can be introduced into a cell for transformation to repress the expression of the polynucleotides (DNA) of (a) to (d) above. Thus, these polynucleotides may suitably be used when repression of the expression of the above polynucleotide (DNA) is preferable.

The phrase "polynucleotide encoding RNA having a nucleotide sequence complementary to the transcript of DNA" as used herein refers to so-called antisense DNA. Antisense technique is known as a method for repressing expression of a particular endogenous gene, and is described in various publications (see e.g., Hirajima and Inoue: New Biochemistry Experiment Course 2 Nucleic Acids IV Gene Replication and Expression (Japanese Biochemical Society Ed., Tokyo Kagaku Dozin Co., Ltd.) pp.319-347, 1993). The sequence of antisense DNA is preferably complementary to all or part of the endogenous gene, but may not be completely complementary as long as it can effectively repress the expression of the gene. The transcribed RNA has preferably 90% or higher, and more preferably 95% or higher complementarity to the transcript of the target gene. The length of the antisense DNA is at least 15 bases or more, preferably 100 bases or more, and more preferably 500 bases or more.

The phrase "polynucteotide encoding RNA that represses DNA expression through RNAi effect" as used herein refers to a polynucleotide for repressing expression of an endogenous gene through RNA interference (RNAi). The term "RNAi" refers to a phenomenon where when double-stranded RNA having a sequence identical or similar to the target gene sequence is introduced into a cell, the expressions of both the introduced foreign gene and the target endogenous gene are repressed. RNA as used herein includes, for example, double-stranded RNA that causes RNA interference of 21 to 25 base length, for example, dsRNA (double strand RNA), siRNA (small interfering RNA) or shRNA (short hairpin RNA). Such RNA may be locally delivered to a desired site with a delivery system such as liposome, or a vector that generates the double-stranded RNA described above may be used for local expression thereof Methods for producing or using such double-stranded RNA (dsRNA, siRNA or shRNA) are known from many publications (see, e.g., Japanese National Phase PCT Laid-open Patent Publication No. 2002-516062; US 2002/085356A; Nature Genetics, 24(2), 180-183, 2000 Feb.; Genesis, 26(4), 240-244, 2000 April, Nature, 407:6802, 319-20, 2002 Sep. 21; Genes & Dev., Vol.16, (8), 948-958, 2002 Apt.15; Proc. Natl. Acad. Sci. USA, 99(8), 5515-5520,2002 Apr. 16; Science, 296(5567), 550-553, 2002 Apr. 19; Proc NatL Acad. Sci. USA, 99:9, 6047-6052, 2002 Apr. 30; Nature Biotechnology, Vol.20 (5), 497-500, 2002 May, Nature Biotechnology, Vol. 20(5), 500-505, 2002 May; Nucleic Acids Res., 30:10, e46,2002 May 15).

The phrase "polynucleotide encoding RNA having an activity of specifically cleaving transcript of DNA" as used herein generally refers to a ribozyme. Ribozyme is an RNA molecule with a catalytic activity that cleaves a transcript of a target DNA and inhibits the function of that gene. Design of ribozymes can be found in various known publications (see, e.g., FEBS Lett. 228: 228, 1988; FEBS Lett. 239: 285, 1988; Nucl. Acids. Res. 17: 7059, 1989; Nature 323: 349, 1986; Nucl. Acids. Res. 19: 6751, 1991; Protein Eng 3: 733, 1990; Nucl. Acids Res. 19: 3875, 1991; Nucl. Acids Res. 19: 5125, 1991; Biochem Biophys Res Commun 186: 1271, 1992). In addition, the phrase "polynucleotide encoding RNA that represses DNA expression through co-supression effect" refers to a nucleotide that inhibits functions of target DNA by "co-supression".

The term "co-supression" as used herein, refers to a phenomenon where when a gene having a sequence identical or similar to a target endogenous gene is transformed into a cell, the expressions of both the introduced foreign gene and the target endogenous gene are repressed. Design of polynucleotides having a co-supression effect can also be found in various publications (see, e.g., Smyth DR: Curr. Biol. 7: R793, 1997, Martienssen R: Curr. Biol. 6: 810, 1996).

### 2. Protein of the present invention

The present invention also provides proteins encoded by any of the polynucleotides (a) to (d) above. A preferred protein of the present invention comprises an amino acid sequence of SEQ ID NO:2 with one to 6 amino acids thereof being deleted, substituted, inserted and/or added, and has an alcohol acetyl transferase activity.

Such protein includes those having an amino acid sequence of SEQ ID NO: 2 with amino acid residues thereof of the number mentioned above being deleted, substituted, inserted and/or added and having an alcohol acetyl transferase activity. In addition, such protein includes those having homology as described above with the amino acid sequence of SEQ ID NO: 2 and having an alcohol acetyl transferase activity.

Such proteins may be obtained by employing site-directed mutation described, for example, in MOLECULAR CLONING 3rd Ed., CURRENT PROTOCOLS IN MOLECULAR BIOLOGY, *Nuc. Acids. Res.,* 10: 6487 (1982), *Proc. Natl. Acad. Sci. USA* 79: 6409 (1982), *Gene* 34: 315 (1985), *Nuc. Acids. Res.,* 13: 4431 (1985), *Proc. Natl. Acad. Sci. USA* 82: 488 (1985).

Deletion, substitution, insertion and/or addition of one to 6 amino acid residues in an amino acid sequence of the protein of the invention means that one to 6 amino acid residues are deleted, substituted, inserted and/or added at any one or more positions in the same amino acid sequence. Two or more types of deletion, substitution, insertion and/or addition may occur concurrently.

Hereinafter, examples of mutually substitutable amino acid residues are enumerated. Amino acid residues in the same group are mutually substitutable. The groups are provided below.

Group A: leucine, isoleucine, norleucine, valine, norvaline, alanine, 2-aminobutanoic acid, methionine, o-methylserine, t-butylglycine, t-butylalanine, cyclohexylalanine; Group B: asparatic acid, glutamic acid, isoasparatic acid, isoglutamic acid, 2-aminoadipic acid, 2-aminosuberic acid; Group C: asparagine, glutamine; Group D: lysine, arginine, ornithine, 2,4-diaminobutanoic acid, 2,3-diaminopropionic acid; Group E: proline, 3-hydroxyproline, 4-hydroxyproline; Group F: serine, threonine, homoserine; and Group G: phenylalanine, tyrosine.

The protein of the present invention may also be produced by chemical synthesis methods such as Fmoc method (fluorenylmethyloxycarbonyl method) and tBoc method (t-butyloxycarbonyl method). In addition, peptide synthesizers available from, for example, Advanced ChemTech, PerkinElmer, Pharmacia, Protein Technology Instrument, Synthecell-Vega, PerSeptive, Shimazu Corp. can also be used for chemical synthesis.

### 3. Vector of the invention and yeast transformed with the vector

The present invention then provides a vector comprising the polynucleotide described above. The vector of the present invention is directed to a vector including any of the polynucleotides described in (a) to (d) above or the polynucleotides described in (j) above. Generally, the vector of the present invention comprises an expression cassette including as components (x) a promoter that can transcribe in a yeast cell; (y) a polynucleotide described in any of (a) to (d) above that is linked to the promoter in sense or antisense direction; and (z) a signal that functions in the yeast with respect to transcription termination and polyadenylation of RNA molecule.

According to the present invention, in order to highly express the protein of the invention described above upon brewing alcoholic beverages (e.g., beer) described below, these polynucleotides are introduced in the sense direction to the promoter to promote expression of the polynucleotide (DNA) described in any of (a) to (d) above. Further, in order to repress the above protein of the invention upon brewing alcoholic beverages (e.g., beer) described below, these polynucleotides are introduced in the antisense direction to the promoter to repress the expression of the polynucleotide (DNA) described in any of (a) to (d) above. In order to repress the above protein of the invention, the polynucleotide may be introduced into vectors such that the polynucleotide of (j) is to be expressed. The target gene (DNA) may also be disrupted to repress the expression of the polynucleotide (DNA) described above or the expression of the protein described above. A gene may be disrupted by adding or deleting one or more bases to or from a region involved in expression of the gene product in the target gene, for example, a coding region or a promoter region, or by deleting these regions entirely. Such disruption of gene may be found in known publications (see, e.g., Proc. Natl. Acad. Sci. USA, 76, 4951(1979), Methods in Enzymology, 101, 202(1983), Japanese Patent Laid-Open Publication No.6-253826).

A vector introduced in the yeast may be any of a multicopy type (YEp type), a single copy type (YCp type), or a chromosome integration type (YIp type). For example, YEp24 (J. R Broach et al., EXPERIMENTAL MANIPULATION OF GENE EXPRESSION, Academic Press, New York, 83, 1983) is known as a YEp type vector, YCp50 (M. D. Rose et al, *Gene* 60: 237, 1987) is known as a YCp type vector, and YIp5 (K Struhl et al., *Proc. Natl. Acad Sci. USA,* 76: 1035, 1979) is known as a YIp type vector, all of which are readily available.

Promoters/terminators for adjusting gene expression in yeast may be in any combination as long as they function in the brewery yeast and they are not influenced by constituents in fermentation broth. For example, a promoter of glyceraldehydes 3-phosphate dehydrogenase gene (TDH3), or a promoter of 3-phosphoglycerate kinase gene (PGK1) may be used. These genes have previously been cloned, described in detail, for example, in M. F. Tuite et al, *EMBO J.,* 1, 603 (1982), and are readily available by known methods.

Since an auxotrophy marker cannot be used as a selective marker upon transformation for a brewery yeast, for example, a geneticm-resistant gene (G418r), a copper-resistant gene (CUP1) (Marin et al., *Proc. Natl. Acad Sci. USA,* 81, 337 1984) or a cerulenin-resistant gene (fas2m, PDR4) (Junji Inokoshi et al, *Biochemistry,* 64, 660, 1992; and Hussain et aL, *Gene,* 101: 149,1991, respectively) may be used

A vector constructed as described above is introduced into a host yeast Examples of the host yeast include any yeast that can be used for brewing, for example, brewery yeasts for beer, wine and sake. Specifically, yeasts such as genus *Saccharomyces* may be used. According to the present invention, a lager brewing yeast, for example, *Saccharomyces pastorianus* W34/70, etc., *Saccharomyces carlsbergensis* NCYC453 or NCYC456, etc., or *Saccharomyces cerevisiae* NBRC1951, NBRC1952, NBRC1953 or NBRC1954, etc., may be used. In addition, whisky yeasts such as *Saccharomyces cerevisiae* NCYC90, wine yeasts such as wine yeasts #1, 3 and 4 from the Brewing Society of Japan, and sake yeasts such as sake yeast #7 and 9 from the Brewing Society of Japan may also be used but not limited thereto. In the present invention, lager brewing yeasts such as *Saccharomyces pastorianus* may be used preferably.

A yeast transformation method may be a generally used known method. For example, methods that can be used include but not limited to an electroporation method (*Meth. Enzym.,* 194: 182 (1990)), a spheroplast method (*Proc. Natl. Acad Sci. USA,* 75: 1929(1978)), a lithium acetate method (*J. Bacteriology,* 153: 163 (1983)), and methods described in *Proc*. *Natl. Acad. Sci. USA,* 75: 1929 (1978), METHODS IN YEAST GENETICS, 2000 Edition: A Cold Spring Harbor Laboratory Course Manual.

More specifically, a host yeast is cultured in a standard yeast nutrition medium (e.g., YEPD medium (Genetic Engineering. Vol. 1, Plenum Press, New York, 117(1979)), etc.) such that OD600 nm will be 1 to 6. This culture yeast is collected by centrifugation, washed and pre-treated with alkali metal ion, preferably lithium ion at a concentration of about 1 to 2 M. After the cell is left to stand at about 30°C for about 60 minutes, it is left to stand with DNA to be introduced (about 1 to 20 µg) at about 30°C for about another 60 minutes. Polyethyleneglycol, preferably about 4,000 Dalton of polyethyleneglycol, is added to a final concentration of about 20% to 50%. After leaving at about 30°C for about 30 minutes, the cell is heated at about 42°C for about 5 minutes. Preferably, this cell suspension is washed with a standard yeast nutrition medium, added to a predetermined amount of fresh standard yeast nutrition medium and left to stand at about 30°C for about 60 minutes. Thereafter, it is seeded to a standard agar medium containing an antibiotic or the like as a selective marker to obtain a transformant

Other general cloning techniques may be found, for example, in MOLECULAR CLONING 3rd Ed., and METHODS IN YEAST GENETICS, A LABORATORY MANUAL (Cold Spring Harbor Laboratory Press, Cold Spring Harbor, NY).

### 4. Method of producing alcoholic beverages according to the present invention and alcoholic beverages produced by the method

The vector of the present invention described above is introduced into a yeast suitable for brewing a target alcoholic product. This yeast can be used to produce a desired alcoholic beverage with enhanced aroma and flavor with an elevated content of ester. In addition, yeasts to be selected by the yeast assessment method of the present invention described below can also be used. The target alcoholic beverages include, for example, but not limited to beer, beer-taste beverages such as sparkling liquor (*happoushu*), wine, whisky, sake and the like. Further, according to the present invention, desired alcoholic beverages with reduced ester level can be produced using brewery yeast in which the expression of the target gene was suppressed, if needed. That is to say, desired kind of alcoholic beverages with controlled (elevated or reduced) level of ester can be produced by controlling (elevating or reducing) production amount of ester using yeasts into which the vector of the present invention was introduced described above, yeasts in which expression of the polynucleotide (DNA) of the present invention described above was suppressed or yeasts selected by the yeast assessment method of the invention described below for fermentation to produce alcoholic beverages.

In order to produce these alcoholic beverages, a known technique can be used except that a brewery yeast obtained according to the present invention is used in the place of a parent strain. Since materials, manufacturing equipment, manufacturing control and the like may be exactly the same as the conventional ones, there is no need of increasing the cost for producing alcoholic beverages with an controlled content of ester. Thus, according to the present invention, alcoholic beverages with excellent aroma and flavor can be produced using the existing facility without increasing the cost.

### 5. Yeast assessment method of the invention

The present invention relates to a method for assessing a test yeast for its ester-producing capability by using a primer or a probe including part or all of the nucleotide sequence of an alcohol acetyl transferase gene having the nucleotide sequence of SEQ ID NO:1 or including a nucleotide sequence complementary to said nucleotide sequence. General techniques for such assessment method is known and is described in, for example, WO01/040514, Japanese Laid-Open Patent PublicationNo. 8-205900 or the like. This assessment method is described in below.

First, genome of a test yeast is prepared. For this preparation, any known method such as Hereford method or potassium acetate method may be used (e.g., METHODS IN YEAST GENETICS, Cold Spring Harbor Laboratory Press, 130 (1990)). Using a primer or a probe designed based on a nucleotide sequence (preferably, ORF sequence) of the alcohol acetyl transferase gene, the existence of the gene or a sequence specific to the gene is determined in the test yeast genome obtained. The primer or the probe may be designed according to a known technique.

Detection of the gene or the specific sequence may be carried out by employing a known technique. In particular, a polynucleotide including part or all of the specific sequence or a polynucleotide including a nucleotide sequence complementary to said nucleotide sequence is used as one primer, while a polynucleotide including part or all of the sequence upstream or downstream from this sequence or a polynucleotide including a nucleotide sequence complementary to said nucleotide sequence, is used as another primer to amplify a nucleic acid of the yeast by a PCR method, thereby determining the existence of amplified products and molecular weight of the amplified products. The number of bases of polynucleotide used for a primer is generally 10 base pairs (bp) or more, and preferably 15 to 25 bp. In general, the number of bases between the primers is suitably 300 to 2000 bp.

The reaction conditions for PCR are not particularly limited but may be, for example, a denaturation temperature of 90 to 95°C, an annealing temperature of 40 to 60°C, an elongation temperature of 60 to 75°C, and the number of cycle of 10 or more. The resulting reaction product may be separated, for example, by electrophoresis using agarose gel to determine the molecular weight of the amplified product. This method allows prediction and assessment of the capability of the yeast to produce ester as determined by whether the molecular weight of the amplified product is a size that contains the DNA molecule of the specific part. In addition, by analyzing the nucleotide sequence of the amplified product, the capability may be predicted and/or assessed more precisely.

Moreover, in the present invention, a test yeast is cultured to measure an expression level of the alcohol acetyl transferase gene having the nucleotide sequence of SEQ ID NO: 1 to assess the test yeast for its ester-producing capability. In measuring an expression level of the alcohol acetyl transferase gene, the test yeast is cultured and then RNA or a protein resulting from the alcohol acetyl transferase gene is quantified. The quantification of mRNA or protein may be carried out by employing a known technique. For example, mRNA may be quantified, by Northern hybridization or quantitative RT-PCR, while protein may be quantified, for example, by Western blotting (CURRENT PROTOCOLS IN MOLECULAR BIOLOGY, John Wiley & Sons 1994-2003).

Furthermore, test yeasts are cultured and expression levels of the alcohol acetyl transferase gene having the nucleotide sequence of SEQ ID NO: 1 are measured to select a test yeast with the gene expression level according to the target capability of producing ester, thereby selecting a yeast favorable for brewing desired alcoholic beverages. In addition, a reference yeast and a test yeast may be cultured so as to measure and compare the expression level of the gene in each of the yeasts, thereby selecting a favorable test yeast. More specifically, for example, a reference yeast and one or more test yeasts are cultured and an expression level of the alcohol acetyl transferase gene having the nucleotide sequence of SEQ ID NO: 1 is measured in each yeast. By selecting a test yeast with the gene expressed higher or lower than that in the reference yeast, a yeast suitable for brewing alcoholic beverages can be selected.

Alternatively, test yeasts are cultured and a yeast with a higher or lower ester-producing capability or with a higher or lower alcohol acetyl transferase activity is selected, thereby selecting a yeast suitable for brewing desired alcoholic beverages.

In these cases, the test yeasts or the reference yeast may be, for example, a yeast introduced with the vector of the invention, a yeast in which an expression of a polynucleotide (DNA) of the invention has been controlled, an artificially mutated yeast or a naturally mutated yeast. The ester-producing capability can be measured by, for example, a method described in Method of J. Am. Soc. Brew. Chem. 49:152-157, 1991. Alcohol acetyl transferase activity can be measured by, for example, a method described in Appl. Microbiol. Biotechnol. 53: 596-600, 2000. The mutation treatment may employ any methods including, for example, physical methods such as ultraviolet irradiation and radiation irradiation, and chemical methods associated with treatments with drugs such as EMS (ethyhmethane sulphonate) and N-methyl-N-nitrosoguanidine (*see, e.g.,* Yasuji Oshima Ed., BIOCHEMISTRY EXPERIMENTS voL 39, *Yeast Molecular Genetic Experiments,* pp. 67-75, JSSP).

In addition, examples of yeasts used as the reference yeast or the test yeasts include any yeasts that can be used for brewing, for example, brewery yeasts for beer, wine, sake and the like. More specifically, yeasts such as genus *Saccharomyces* may be used (*e.g., S. pastorianus. S. cerevisiae,* and *S*. *carlsbergensis).* According to the present invention, a lager brewing yeast, for example, *Saccharomyces pastorianus* W34/70; *Saccharomyces carlsbergensis* NCYC453 or NCYC456; or *Saccharomyces cerevisiae* NBRC1951, NBRC1952, NBRC1953 or NBRC1954, etc., may be used Further, wine yeasts such as wine yeasts #1, 3 and 4 from the Brewing Society of Japan; and sake yeasts such as sake yeast #7 and 9 from the Brewing Society of Japan may also be used but not limited thereto. In the present invention, lager brewing yeasts such as *Saccharomyces pastorianus* may preferably be used The reference yeast and the test yeasts may be selected from the above yeasts in any combination

### EXAMPLES

Hereinafter, the present invention will be described in more detail with reference to working examples. The present invention, however, is not limited to the examples described below.

### Example 1: Cloning of Alcohol acetyl transferase Gene (nonScTF2)

A specific novel alcohol acetyl transferase gene (nonScATF2) (SEQ ID NO: 1) from a lager brewing yeast were found, as a resuh of a search utilizing the comparison database described in Japanese Patent Laid-Open Publication No. 2004-283169. Based on the acquired nucleotide sequence information, primers nonScATF2-for (SEQ ID NO: 3) and nonScATF2_rv (SEQ ID NO: 4) were designed to amplify the full-length genes, respectively. PCR was carried out using chromosomal DNA of a genome sequencing strain, *Saccharomyces pastorianus* Weienstephan 34/70 strain, also abbreviated to "W34/70 strain", as a template to obtain DNA fragments (about 0.7 kb) including the full-length gene of nonScATF2.

The thus-obtained nonScATF2 gene fragment was inserted into pCR2.1-TOPO vector (Invitrogen) by TA cloning. The nucleotide sequences of nonScATF2 gene were analyzed according to Sanger's method (F. Sanger, *Science,* 214: 1215, 1981) to confirm the nucleotide sequence.

### Example 2: Analysis of Expression of monScATF2 Gene during Beer Fermentation Test

A beer fermentation test was conducted using a lager brewing yeast, *Saccharomyces pastorianus* W34/70 strain and then mRNA extracted from yeast cells during fermentation was analyzed by a DNA microarray.

| | |
|---|---|
| Wort extract concentration | 12.69% |
| Wort content | 70 L |
| Wort dissolved oxygen concentration | 8.6 ppm |
| Fermentation temperature | 15°C |
| Yeast pitching rate | 12.8×10⁶ cells/mL |

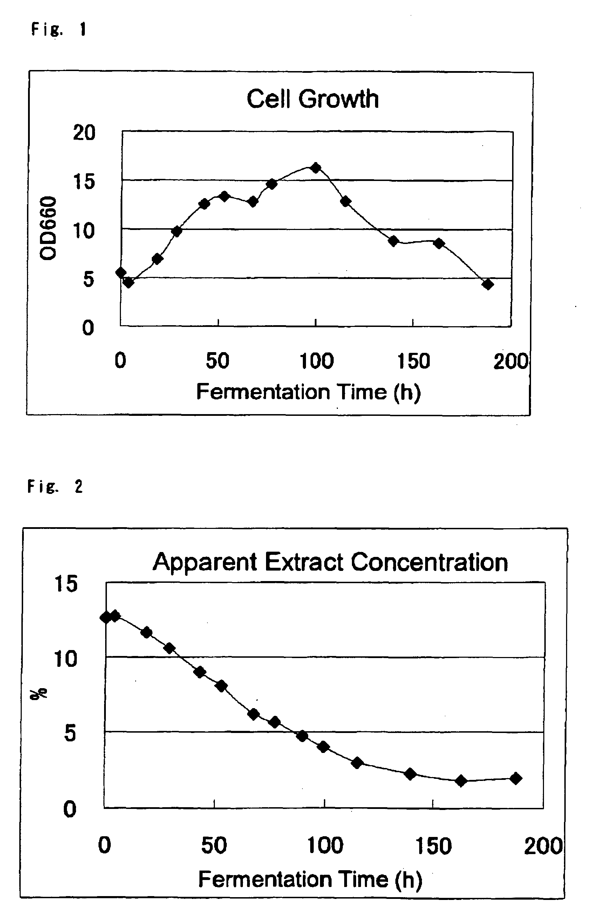

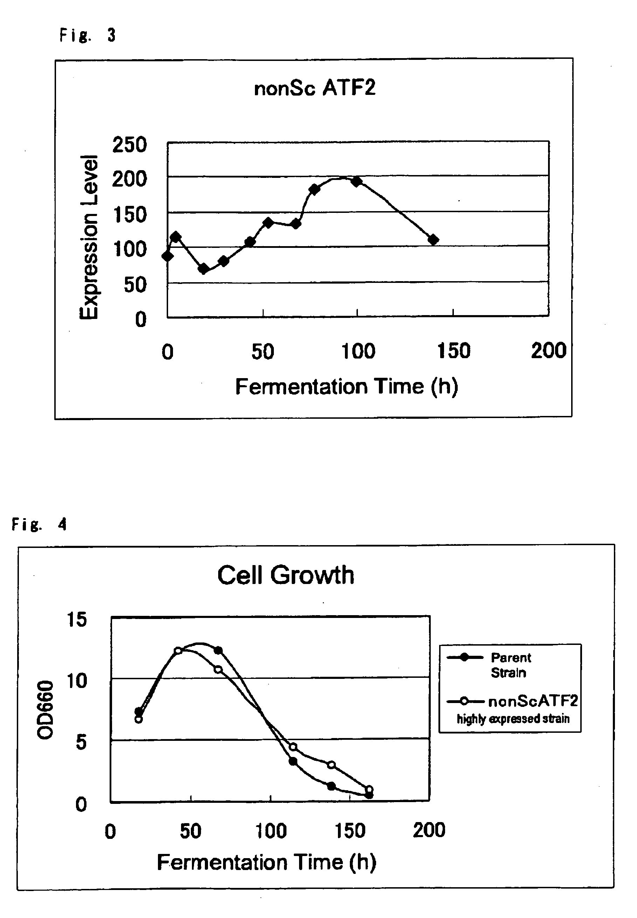

Sampling of fermentation liquor was performed with time, and variation with time of yeast growth amount (Fig. 1) and apparent extract concentration (Fig. 2) was observed. Simultaneously, sampling of yeast cells was performer, and the prepared mRNA was subjected to be biotin-labeled and was hybridized to a beer yeast DNA microarray. The signal was detected using GCOS; GeneChip Operating Software 1.0 (manufactured by Affymetrix Co.). Expression pattern of nonScATF2 gene is shown in Figure 3. As a result, it was confirmed that nonScATF2 gene was expressed in the general beer fermentation.

### Example 3: Construction of nonScATF2 Gene Highly Expressed Strain

The nonScATF2/pCR2.1-TOPO described in Example 1 was digested using the restriction enzymes SacI and NotI so as to prepare a DNA fragment containing the entire length of the protein-encoding region. This fragment was ligated to pYCGPYNot treated with the restriction enzymes SacI and NotI, thereby constructing the nonScATF2 high expression vector nonScATF2/pYCGPYNot. pYCGPYNot is the YCp-type yeast expression vector. The inserted gene is highly expressed by the pyruvate kinase gene PYKI promoter. The geneticin-resistant gene G418^{r} is included as the selection marker in the yeast, and the ampicillin-resistant gene Amp^{r} is included as the selection marker in *Escherichia coli.*

Using the high expression vector prepared by the above method, the strain *Saccharomyces pasteurianus* Weihenstephaner 34/70 was transformed by the method described in Japanese Patent Laid-open Publication No. H7-303475. The transformant was selected in a YPD plate culture (1% yeast extract, 2% polypeptone, 2% glucose, 2% agar) containing 300 mg/L of geneticin.

It should be noted that strain W34/70-2Δe is a strain W34/70-2, which is a spore clone of Saccharomyces pastorianus Weihenstephan W34/70 in which gene non-ScEHT1, which encodes non-ScEht1p, an alcohol acetyl transferase characteristic to brewer's yeast, has been destroyed. Disruption of the nonScEHT1 gene was carried out in accordance with a method described in the literature (Goldstein et aL, Yeast, 15, 1541 (1999)). Fragments for gene disruption were prepared by PCR using plasmids containing a drug resistance marker (pFA6a(G418r), pAG25(nat1), pAG32(hph)) as templates. Primers consisting of nonScEHT1_delta_for (SEQ ID NO. 7) and nonScETH1_deha_rv (SEQ ID NO. 8) were used for the PCR primers. A spore clone (W34/70-2) isolated from brewer's yeast Saccharomyces pastorianus strain W34/70 was transformed with the fragments for gene disruption prepared as described above. Transformtion was carried out according to the method described in Japanese Patent Laid-open Publication No. H07-303475, and transformants were selected on YPD plate medium (1% yeast extract, 2% polypeptone, 2% glucose, 2% agar) containing geneticin at 300 mg/L, nourseothricin at 50 mg/L or hygromycin B at 200 mg/L.

### Example 4: Analysis of Amounts of Ester Formed in Beer Test Brewing

A fermentation test was conducted under the following conditions using the parent strain and the nonScATF2 highly expressing strain obtained in Example 3.
Wort extract concentration: 12%
Wort volume: 1 L
Wort dissolved oxygen concentration: 9.5 ppm
Fermentation temperature: 15°C
yeast pitching rate 5 g/L

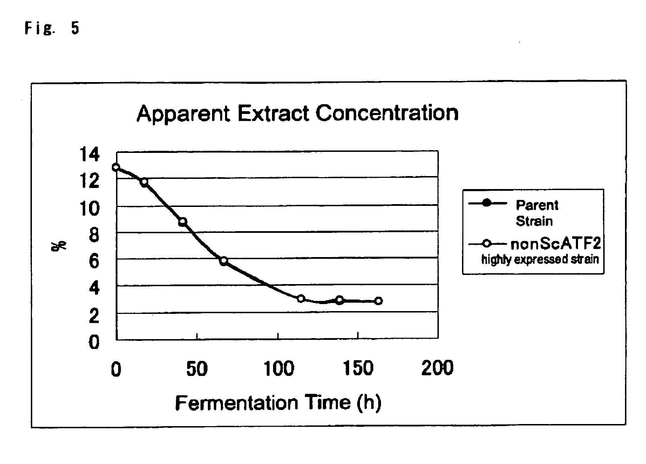

The fermentation broth was sampled over time to investigate the time-based changes in yeast growth (OD660) (Fig. 4) and extract consumption (Fig. 5). Quantification of higher alcohol and extract concentrations at completion of fermentation was carried out using head space gas chromatography (J. Am. Soc. Brew. Chem. 49:152-157, 1991).

According to Table 1, the amount of ethyl acetate formed at completion of fermentation was 37.0 ppm for the nonScATF2 highly expressing strain in contrast to 33.0 ppm for the parent strain. The amount of isoamyl alcohol formed was 3.8 ppm for the nonScATF2 highly expressing strain in contrast to 2.9 ppm for the parent strain. On the basis of these results, the amounts of ethyl acetate and isoamyl alcohol formed by the mnScATF2 highly expressing strain were clearly demonstrated to increased by 12 to 31%.

**Table 1**

| | Parent Strain | NonScATF2 Highly | |
|---|---|---|---|
| | | Expressing Strain | |
| Ethyl acetate | 33.0 | 37.0 | (112%) |
| Isoamyl alcohol | 2.9 | 3.8 | (131%) |

| | | | |
|---|---|---|---|
| Unit: ppm Values in parentheses indicate relative values versus the parent strain. | | | |

### Example 5: Disruption of nonScATF2 Gene

Fragments for gene disruption are prepared by PCR using plasmids containing a drug resistance marker (pFA6a(G418r), pAG25(nat1), pAG32(hph)) as templates in accordance with a method described in the literature (Goldstein et al., Yeast, 15, 1541 (1999)). Primers consisting of nonScATF2_delta_for (SEQ ID NO. 5) and nonScATF2_delta_rv (SEQ ID NO. 6) are used for the PCR primers.

Brewer's yeast Saccharomyces pastorianus strain W34/70 or a spore clone (W34/70-2) isolated from brewer's yeast Saccharomyces pastorianus strain W34/70 is transformed with the fragments for gene disruption prepared as described above. Transformation is carried out according to the method described in Japanese Patent Laid-open Publication No. H07-303475, and transformants are selected on YPD plate medium (1% yeast extract, 2% polypeptone, 2% glucose, 2% agar) containing geneticin at 300 mg/L, nourseothricin at 50 mg/L or hygromycin B at 200 mg/L.

### Example 6: Analysis of Amounts of Ester Formed in Beer Test Brewing

A fermentation test is conducted under the following conditions using the parent strain and the nonScATF2 disrupted strain obtained in Example 5.
Wort extract concentration: 12%
Wort volume: 1 L
Wort dissolved oxygen concentration: 10 ppm
Fermentation temperature: 15°C
yeast pitching rate: 5 g/L

The fermentation broth is sampled over time to investigate the time-based changes in yeast growth (OD660) and extract consumption. Quantification of ester concentration at completion of fermentation is carried out according to the method described in J. Am. Soc. Brew. Chem. 49:152-157, 1991 using head space gas chromatography.

### Industrial Applicability

According to the alcoholic beverage production method of the present invention, alcoholic beverages having superior aroma and flavor can be produced by increasing the content of esters which impart a florid aroma to products. In addition, in the case of malt beverages such as beer, for which an excessively high ester content is not preferred, alcoholic beverages having a more desirable aroma and flavor can be produced by decreasing the amount of ester contained therein. This application claims benefit of Japanese Patent Application Nos. 2005-266068 filed September 13, 2005 and 2006-200892 filed July 24, 2006.

## Claims

1. A polynucleotide selected from the group consisting of:
(a) a polynucleotide comprising a polynucleotide consisting of the nucleotide sequence of SEQ ID NO:1;
(b) a polynucleotide comprising a polynucleotide encoding a protein consisting of the amino acid sequence of SEQ ID NO:2;
(c) a polynucleotide comprising a polynucleotide encoding a protein consisting of the amino acid sequence of SEQ ID NO:2 with one to 6 amino acids thereof being deleted, substituted, inserted and/or added, and having an alcohol acetyl transferase activity; and
(d) a polynucleotide comprising a polynucleotide encoding a protein having an amino acid sequence having 97% or higher identity with the amino acid sequence of SEQ ID NO:2, and having an alcohol acetyl transferase activity.

2. The polynucleotide of Claim 1 selected from the group consisting of:
(g) a polynucleotide comprising a polynucleotide encoding a protein consisting of the amino acid sequence of SEQ ID NO: 2, or encoding an amino acid sequence of SEQ ID NO: 2 wherein 1 to 2 amino acids thereof is deleted, substituted, inserted, and/or added, and wherein said protein has an alcohol acetyl transferase activity; and
(h) a polynucleotide comprising a polynucleotide encoding a protein having 99% or higher identity with the amino acid sequence of SEQ ID NO: 2, and having an alcohol acetyl transferase activity.

3. The polynucleotide of Claim 1 comprising a polynucleotide consisting of the nucleotide sequence of SEQ ID NO: 1.

4. The polynucleotide of Claim 1 comprising a polynucleotide encoding a protein consisting of the amino acid sequence of SEQ ID NO: 2.

5. The polynucleotide of any one of Claims 1 to 4, wherein the polynucleotide is DNA.

6. A polynucleotide encoding RNA of a nucleotide sequence complementary to a transcript of the polynucleotide (DNA) according to Claim 5.

7. A protein encoded by the polynucleotide of any one of Claims 1 to 5.

8. A vector comprising the polynucleotide of any one of Claims 1 to 5.

9. A vector comprising the polynucleotide of Claim 6.

10. A yeast comprising the vector of Claim 8 or 9.

11. The yeast of Claim 10, wherein an ester-producing ability is increased by introducing the vector of Claim 8.

12. A yeast, wherein an expression of the polynucleotide (DNA) of Claim 5 is repressed by introducing the vector of Claim 9, or by disrupting the polynucleotide (DNA) of Claim 5.

13. The yeast of Claim 11, wherein an ester-producing ability is increased by increasing an expression level of the protein of Claim 7.

14. A method for producing an alcoholic beverage by using the yeast of any one of Claims 10 to 13.

15. The method for producing an alcoholic beverage of Claim 14, wherein the brewed alcoholic beverage is a malt beverage.

16. The method for producing an alcoholic beverage of Claim 14, wherein the brewed alcoholic beverage is wine.

17. A method for assessing a test yeast for its ester-producing capability, comprising using a primer or a probe including part or all of the nucleotide sequence of an alcohol acetyl transferase gene having the nucleotide sequence of SEQ ID NO: 1 or including a nucleotide sequence complementary to said nucleotide sequence.

18. A method for assessing a test yeast for its ester-producing capability, comprising: culturing a test yeast; and measuring an expression level of an alcohol acetyl transferase gene having the nucleotide sequence of SEQ ID NO: 1.

19. A method for selecting a yeast, comprising: culturing test yeasts; quantifying the protein according to Claim 7 or measuring an expression level of an alcohol acetyl transferase gene having the nucleotide sequence of SEQ ID NO: 1; and selecting a test yeast having said protein amount or said gene expression level according to a target capability of producing ester.

20. The method for selecting a yeast according to Claim 19, comprising: culturing a reference yeast and test yeasts; measuring an expression level of an alcohol acetyl transferase gene having the nucleotide sequence of SEQ ID NO: 1 in each yeast; and selecting a test yeast having the gene expressed higher or lower than that in the reference yeast.

21. The method for selecting a yeast according to Claim 19, comprising: culturing a reference yeast and test yeasts; quantifying the protein according to Claim 7 in each yeast; and selecting a test yeast having said protein for a larger or smaller amount than that in the reference yeast.

22. A method for producing an alcoholic beverage comprising: conducting fermentation for producing an alcoholic beverage using the yeast according to any one of Claims 10 to 13 or a yeast selected by the method according to any one of Claims 19 to 21; and adjusting the production amount of ester.

## Patentansprüche

1. Polynucleotid ausgewählt aus der Gruppe bestehend aus:
(a) einem Polynucleotid umfassend ein Polynucleotid bestehend aus der Nucleotidsequenz dargestellt durch SEQ ID NO:1;
(b) einem Polynucleotid umfassend ein Polynucleotid, das ein Protein codiert, das aus der Aminosäuresequenz dargestellt durch SEQ ID NO:2 besteht;
(c) einem Polynucleotid umfassend ein Polynucleotid, das ein Protein codiert, das aus der Aminosäuresequenz dargestellt durch SEQ ID NO:2 besteht, wobei eine bis 6 Aminosäuren davon deletiert, substituiert, inseriert und/oder hinzugefügt sind und wobei das Protein eine Alkoholacetyltransferaseaktivität hat; und
(d) einem Polynucleotid umfassend ein Polynucleotid, das ein Protein codiert, das eine Aminosäuresequenz hat, die eine 97%ige oder höhere Identität mit der Aminosäuresequenz dargestellt durch SEQ ID NO:2 hat und wobei das Protein eine Alkoholacetyltransferaseaktivität hat.

2. Polynucleotid gemäß Anspruch 1 ausgewählt aus der Gruppe bestehend aus:
(g) einem Polynucleotid umfassend ein Polynucleotid, das ein Protein codiert, das aus der Aminosäuresequenz dargestellt durch SEQ ID NO:2 besteht, oder das eine Aminosäuresequenz von SEQ ID NO:2 codiert, wobei 1 bis 2 Aminosäuren davon deletiert, substituiert, inseriert und/oder hinzugefügt sind und wobei das Protein eine Alkoholacetyltransferaseaktivität hat; und
(h) einem Polynucleotid, umfassend ein Polynucleotid, das ein Protein codiert, das eine 99%ige oder höhere Identität mit der Aminosäuresequenz dargestellt durch SEQ ID NO:2 hat und wobei das Protein eine Alkoholacetyltransferaseaktivität hat.

3. Polynucleotid gemäß Anspruch 1, umfassend ein Polynucleotid, das aus der Nucleotidsequenz, dargestellt durch SEQ ID NO:1, besteht.

4. Polynucleotid gemäß Anspruch 1, umfassend ein Polynucleotid, das ein Protein codiert, das aus der Aminosäuresequenz, dargestellt durch SEQ ID NO:2, besteht.

5. Polynucleotid gemäß einem der Ansprüche 1 bis 4, wobei das Polynucleotid DNA ist.

6. Polynucleotid, das RNA von einer Nucleotidsequenz codiert, die komplementär zu einem Transkript des Polynucleotids (DNA) gemäß Anspruch 5 ist.

7. Protein, das durch das Polynucleotid gemäß einem der Ansprüche 1 bis 5 codiert wird.

8. Vektor umfassend das Polynucleotid gemäß einem der Ansprüche 1 bis 5.

9. Vektor umfassend das Polynucleotid gemäß Anspruch 6.

10. Hefe umfassend den Vektor gemäß Anspruch 8 oder 9.

11. Hefe gemäß Anspruch 10, wobei eine Fähigkeit Ester herzustellen gesteigert ist durch das Einführen des Vektors gemäß Anspruch 8.

12. Hefe, in der die Expression des Polynucleotids (DNA) gemäß Anspruch 5 unterdrückt ist durch das Einführen des Vektors gemäß Anspruch 9 oder durch die Unterbrechung des Polynucleotids (DNA) gemäß Anspruch 5.

13. Hefe gemäß Anspruch 11, wobei eine Fähigkeit Ester herzustellen gesteigert ist durch das Erhöhen eines Expressionsspiegels des Proteins gemäß Anspruch 7.

14. Verfahren zur Herstellung eines alkoholischen Getränks durch Verwendung der Hefe gemäß einem der Ansprüche 10 bis 13.

15. Verfahren zur Herstellung eines alkoholischen Getränks gemäß Anspruch 14, wobei das gebraute alkoholische Getränk ein Malzgetränk ist.

16. Verfahren zur Herstellung eines alkoholischen Getränks gemäß Anspruch 14, wobei das gebraute alkoholische Getränk Wein ist.

17. Verfahren zur Bewertung der Fähigkeit einer Testhefe Ester herzustellen, umfassend die Verwendung eines Primers oder einer Sonde, umfassend einen Teil oder die Gesamtheit der Nucleotidsequenz eines Alkoholacetyltransferasegens, das die Nucleotidsequenz dargestellt durch SEQ ID NO:1 hat, oder umfassend eine Nucleotidsequenz, die komplementär zu der Nucleotidsequenz ist.

18. Verfahren zur Bewertung der Fähigkeit einer Testhefe Ester herzustellen, umfassend: Züchten einer Testhefe; und Messen eines Expressionsspiegels eines Alkoholacetyltransferasegens, das die Nucleotidsequenz dargestellt durch SEQ ID NO:1 hat.

19. Verfahren zum Auswählen einer Hefe, umfassend: Züchten von Testhefen; Quantifizieren des Proteins gemäß Anspruch 7 oder Messen eines Expressionsspiegels eines Alkoholacetyltransferasegens, das die Nucleotidsequenz dargestellt durch SEQ ID NO:1 hat; und Auswählen einer Testhefe, die diese Proteinmenge oder diesen Genexpressionsspiegel hat, entsprechend der Zielfähigkeit Ester herzustellen.

20. Verfahren zum Auswählen einer Hefe gemäß Anspruch 19, umfassend: Züchten einer Referenzhefe und von Testhefen; Messen eines Expressionsspiegels eines Alkoholacetyltransferasegens, das die Nucleotidsequenz dargestellt durch SEQ ID NO:1 hat, in jeder Hefe; und Auswählen einer Testhefe, die das Gen höher oder niedriger als in der Referenzhefe exprimiert.

21. Verfahren zum Auswählen einer Hefe gemäß Anspruch 19, umfassend: Züchten einer Referenzhefe und von Testhefen; Quantifizieren des Proteins gemäß Anspruch 7 in jeder Hefe, und Auswählen einer Testhefe, die das Protein in einer größeren oder geringeren Menge hat als die Referenzhefe.

22. Verfahren zum Herstellen eines alkoholischen Getränks, umfassend: Durchführen einer Fermentation zur Herstellung eines alkoholischen Getränks unter Verwendung der Hefe gemäß einem der Ansprüche 10 bis 13 oder einer Hefe ausgewählt durch das Verfahren gemäß einem der Ansprüche 19 bis 21; und Anpassen der produzierten Menge an Ester.

## Revendications

1. Polynucléotide choisi parmi le groupe consistant en:
(a) un polynucléotide comprenant un polynucléotide consistant en la séquence nucléotidique de SEQ ID NO: 1;
(b) un polynucléotide comprenant un polynucléotide codant pour une protéine consistant en la séquence d'acides aminés de SEQ ID NO: 2;
(c) un polynucléotide comprenant un polynucléotide codant pour une protéine consistant en la séquence d'acides aminés de SEQ ID NO: 2 avec un à 6 de ses acides aminés étant supprimés, substitués, insérés et/ou ajoutés, et ayant une activité d'alcool acétyl transférase ; et
(d) un polynucléotide comprenant un polynucléotide codant pour une protéine ayant une séquence d'acides aminés ayant 97 % ou plus d'identité avec la séquence d'acides aminés de SEQ ID NO: 2, et ayant une activité d'alcool acétyl transférase.

2. Polynucléotide selon la revendication 1 choisi parmi le groupe consistant en:
(g) un polynucléotide comprenant un polynucléotide codant pour une protéine consistant en la séquence d'acides aminés de SEQ ID NO: 2, ou codant pour une séquence d'acides aminés de SEQ ID NO: 2 où 1 à 2 de ses acides aminés est supprimé, substitué, inséré, et/ou ajouté, et où ladite protéine possède une activité d'alcool acétyl transférase; et
(h) un polynucléotide comprenant un polynucléotide codant pour une protéine ayant 99 % ou plus d'identité avec la séquence d'acides aminés de SEQ ID NO: 2, et ayant une activité d'alcool acétyl transférase.

3. Polynucléotide selon la revendication 1 comprenant un polynucléotide consistant en la séquence nucléotidique de SEQ ID NO: 1.

4. Polynucléotide selon la revendication 1 comprenant un polynucléotide codant pour une protéine consistant en la séquence d'acides aminés de SEQ ID NO: 2.

5. Polynucléotide selon l'une quelconque des revendications 1 à 4, où le polynucléotide est de l'ADN.

6. Polynucléotide codant pour l'ARN d'une séquence nucléotidique complémentaire d'un produit de transcription du polynucléotide (ADN) selon la revendication 5.

7. Protéine codée par le polynucléotide selon l'une quelconque des revendications 1 à 5.

8. Vecteur comprenant le polynucléotide selon l'une quelconque des revendications 1 à 5.

9. Vecteur comprenant le polynucléotide selon la revendication 6.

10. Levure comprenant le vecteur selon la revendication 8 ou 9.

11. Levure selon la revendication 10, où une capacité à produire un ester est augmentée par l'introduction du vecteur selon la revendication 8.

12. Levure, où une expression du polynucléotide (ADN) selon la revendication 5 est réprimée en introduisant le vecteur selon la revendication 9, ou par dislocation du polynucléotide (ADN) selon la revendication 5.

13. Levure selon la revendication 11, où une capacité à produire un ester est augmentée en augmentant un niveau d'expression de la protéine selon la revendication 7.

14. Procédé de production d'une boisson alcoolique en utilisant la levure selon l'une quelconque des revendications 10 à 13.

15. Procédé de production d'une boisson alcoolique selon la revendication 14, où la boisson alcoolique brassée est une boisson à base de malt.

16. Procédé de production d'une boisson alcoolique selon la revendication 14, où la boisson alcoolique brassée est du vin.

17. Procédé destiné à évaluer une levure d'essai en ce qui concerne sa capacité à produire un ester, consistant à utiliser une amorce ou une sonde comprenant une partie ou l'ensemble de la séquence nucléotidique d'un gène d'alcool acétyl transférase ayant la séquence nucléotidique de SEQ ID NO: 1 ou incluant une séquence nucléotidique complémentaire de ladite séquence nucléotidique.

18. Procédé destiné à évaluer une levure d'essai en ce qui concerne sa capacité à produire un ester, consistant à : mettre en culture une levure d'essai; et mesurer un niveau d'expression d'un gène d'alcool acétyl transférase ayant la séquence nucléotidique de SEQ ID NO: 1.

19. Procédé de sélection d'une levure, consistant à: mettre en culture des levures d'essai ; quantifier la protéine selon la revendication 7 ou mesurer un niveau d'expression d'un gène d'alcool acétyl transférase ayant la séquence nucléotidique de SEQ ID NO: 1; et choisir une levure d'essai ayant ladite quantité de protéines ou ledit niveau d'expression du gène selon une capacité cible de production d'ester.

20. Procédé de sélection d'une levure selon la revendication 19, consistant à: mettre en culture une levure de référence et des levures d'essai; mesurer un niveau d'expression d'un gène d'alcool acétyl transférase ayant la séquence nucléotidique de SEQ ID NO: 1 dans chaque levure; et sélectionner une levure d'essai ayant le gène plus fortement ou plus faiblement exprimé que dans la levure de référence.

21. Procédé de sélection d'une levure selon la revendication 19, consistant à: mettre en culture une levure de référence et des levures d'essai; quantifier la protéine selon la revendication 7 dans chaque levure; et sélectionner une levure d'essai ayant ladite protéine en une plus grande ou plus petite quantité que dans la levure de référence.

22. Procédé de production d'une boisson alcoolique consistant à: réaliser une fermentation pour produire une boisson alcoolique utilisant la levure selon l'une quelconque des revendications 10 à 13 ou une levure choisie parmi le procédé selon l'une quelconque des revendications 19 à 21 ; et ajuster la quantité de production d'ester.
